# EUROPEAN PATENT SPECIFICATION

(11) **EP 0 544 760 B1**
(45) Date of publication and mention of the grant of the patent: **02.05.1997**
(21) Application number: 91915307.2
(22) Date of filing: 23.08.1991
(51) Int. Cl.: A61K 31/44

(54) **PHARMACEUTICAL COMPOSITIONS CONTAINING 5-DIFLUOROMETHOXY-2- (3,4-DIMETHOXY-2-PYRIDYL) METHYLSULFINYL] BENZIMIDAZOLE AND AN ANTI-HELICOBACTER AGENT FOR THE TREATMENT OF GASTROINTESTINAL DISORDERS**
PHARMAZEUTISCHE ZUSAMMENSETZUNGEN DIE 5-DIFLUORMETHOXY-2-[3,4-DIMETHOXY-2-PYRIDYL)METHYLSULFINYL]-1H-BENZIMIDAZOL UND EIN ANTI-HELIOBACTERMITTEL ENTHALTEN ZUR BEHANDLUNG VON MAGEN-DARM KRANKHEITEN
COMPOSITIONS PHARMACEUTIQUES CONTENANT DU 5-DIFLUOROMETHOXY-2- (3,4-DIMETHOXY-2-PYRIDYLE) METHYLSULFINYLE]BENZIMIDAZOLE ET UN AGENT ANTI-HELICOBACTER POUR LE TRAITEMENT DES TROUBLES GASTRO-INTESTINAUX

(30) Priority: 24.08.1990 GB 9018603
(43) Date of publication of application: 09.06.1993
(73) Proprietor: BYK GULDEN LOMBERG CHEMISCHE FABRIK GMBH, D-78403 Konstanz (DE)
(72) Inventor: PARSONS, Michael, E., Welwyn Hertfordshire AL6 9AR (GB)
(86) International application number: GB9101427
(87) International publication number: WO9203135

(56) References cited:
- EP-A- 0 282 131
- WO-A-89/03219
- WO-A-90/09175
- Antimicrobial Agents and Chemotherapy, vol. 28, no. 6, December 1985; C.A.M. McNulty et al.: "Susceptibility of clinical isolates of campylobacter pylorides to 11 antimicrobial agents", pages 837-838, see abstract; page 837, column 1, paragraph 2; page 838, table 1, paragraphs 2 and 3

## Description

The present invention relates to pharmaceutical compositions and their use in treating or preventing gastrointestinal disorders, in particular disorders caused or exacerbated by helicobacter infection and secreted gastric acid.

The compositions of the invention comprise a helicobacter-inhibiting anti-microbial agent and a compound of formula (1): 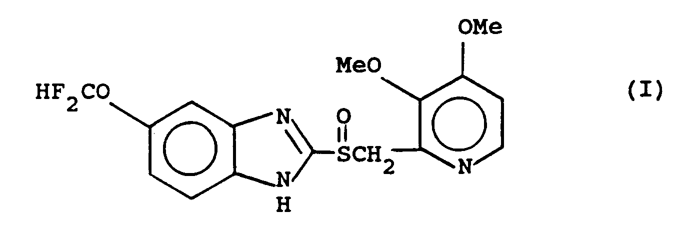 or a pharmaceutically acceptable salt thereof.

The present invention further relates to the use of a helicobacter-inhibiting anti-microbial agent and a compound of formula (I) or a pharmaceutically acceptable salt thereof in treating or preventing gastrointestinal disorders in mammals, in particular humans. This use may involve either concurrent or non-concurrent administration of the helicobacter-inhibiting anti-microbial agent and a compound of formula (I) or a pharmaceutically acceptable salt thereof.

The term "helicobacter-inhibiting anti-microbial agent" means any natural, synthetic, or semi-synthetic compound or mixture thereof which is effective in eradicating helicobacter pylori organisms (formerly known as campylobacter pylori organisms).

Such helicobacter-like organisms and helicobacter-inhibiting anti-microbial agents, as well as the various in vitro and in vivo assays used to determine the effectiveness of such agents, have been described in EP-A-0 282 131.

Suitable anti-microbial agents include antibiotics, and bismuth salts such as bismuth subcitrate or bismuth subsalicylate.

Antibiotics are the preferred helicobacter-inhibiting anti-microbial agents useful herein. Specific examples of such helicobacter-inhibiting anti-microbial agents include penicillin, mezlocillin, ampicillin, amoxicillin, cefalothin, cefoxitin, cefotaxime, imipenem, gentamicin, amikacin, erythromycin, ciprofloxacin, tetracyclines, metronidazole, cephalosporins, and combinations thereof. The preferred helicobacter-inhibiting anti-microbial agent is amoxicillin. The more preferred helicobacter-inhibiting agent is amoxicillin in combination with metronidazole. The most preferred helicobacter-inhibiting anti-microbial agent is a combination of amoxicillin, metronidazole and a bismuth salt as disclosed in WO 89/03219.

The compound of formula (I) can be prepared using the procedures described in EP 0 166 287-B. In particular, the compound of formula (I) in the form of its sodium salt is preferred.

The compositions of the present invention may be used in therapy to treat gastrointestinal diseases caused or exacerbated by helicobacter infection and secreted gastric acid. For example, they may be used to treat duodenal and gastric ulcer disease, in particular having a positive effect in lowering the relapse rate of such diseases compared to the relapse rate observed by treatment with a compound of structure (I) alone.

The use of the present invention in therapy comprises administering the helicobacter-inhibiting anti-microbial agent and a compound of formula (I) or a pharmaceutically acceptable salt thereof either concurrently or non-concurrently. Concurrently means that the two agents are administered within 24 hours or less of each other, preferably within about 12 hours of each other, more preferably within about 1 hour of each other and most preferably within about 5 minutes of each other; and includes co-administration of the agents by administering a composition of the present invention. The term non-concurrently means that the two agents are administered more than 24 hours apart.

In a still further aspect, the present invention provides a method of treatment of gastrointestinal diseases caused or exacerbated by H.pylori infection and elevated levels of gastric acid which comprises administering to a subject in need thereof, an effective amount of a compound of structure (I) or a pharmaceutically acceptable salt thereof and a helicobacter-inhibiting anti-microbial agent.

In therapeutic use the anti-microbial agent and the compound of formula (I) or a pharmaceutically acceptable salt thereof can be administered separately in a standard pharmaceutical composition, or together in a single composition.

Standard compositions can be prepared by techniques well-known in the art of pharmacy, for example as described in EP-0 166 287-B.

The daily dose regimen for an adult patient involves administering the helicobacter-inhibiting anti-microbial agent in an amount from 1mg to 10000mg. The specific quantity depends on the particular anti-microbial agent used. For example, penicillins such as amoxicillin are administered in an amount of from about 500mg to about 3000mg per day, preferably from about 750mg to about 1500mg per day; bismuth salts such as bismuth subcitrate and bismuth subsalicylate are administered in an amount of from about 5mg to about 5000mg per day, preferably from about 50mg to about 250mg per day.

The daily dosage regimen for an adult patient for the compound of formula (I) or a pharmaceutically acceptable salt thereof involves administering from about 0.7mg to about 1400mg per day calculated as the free base. Preferably the dose is from about 3.5mg to about 350mg per day calculated as the free base and most preferably from about 7mg to about 100mg per day calculated as the free base.

Suitably the anti-microbial agent and the compound of formula (I) or a pharmaceutically acceptable salt thereof can be administered together in several unit doses, preferably 1-4 times per day. In the case of parenteral treatment lower doses can generally be used. Suitably the compounds will be administered for a period of continuous therapy, for example a week or more.

In a still further aspect, the present invention provides the use of a helicobacter-inhibiting antimicrobial agent and a compound of formula (I) or a pharmaceutically acceptable salt thereof in the manufacture of a medicament for treating or preventing gastrointestinal disorders, in particular ulcer relapse. It is to be understood that when used herein, 'medicament' shall be taken to refer to a composition comprising both the helicobacter-inhibiting anti-microbial agent and the compound of formula (I) or a pharmaceutically acceptable salt thereof, or a medicament pack comprising the two active ingredients as discrete separate dosage forms.

## Claims

1. A pharmaceutical composition comprising a helicobacter-inhibiting anti-microbial agent and a compound of formula (I): 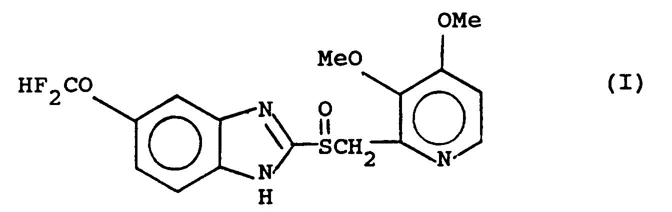 or a pharmaceutically acceptable salt thereof, and a pharmaceutically acceptable carrier.

2. A pharmaceutical composition according to claim 1 wherein the compound of formula (I) is in the form of its sodium salt.

3. A pharmaceutical composition according to claim 2 wherein the helicobacter-inhibiting anti-microbial agent is amoxicillin.

4. A pharmaceutical composition according to claim 2 wherein the helicobacter-inhibiting anti-microbial agent is amoxicillin and metronidazole.

5. A pharmaceutical composition according to any one of claims 2-4 wherein the helicobacter-inhibiting anti-microbial agent further comprises a pharmaceutically acceptable bismuth salt.

6. A pharmaceutical composition according to claim 1 wherein the helicobacter-inhibiting anti-microbial agent is a pharmaceutically acceptable bismuth salt.

7. A pharmaceutical composition according to any one of claims 1-6 for use in therapy.

8. A pharmaceutical composition according to any one of claims 1-6 for use in treating or preventing gastrointestinal disorders.

9. The use of a helicobacter-inhibiting anti-microbial agent and a compound of formula (I) or a pharmaceutically acceptable salt thereof in the manufacture of a medicament for treating or preventing gastrointestinal disorders.

10. The use of a helicobacter-inhibiting anti-microbial agent and a compound of formula (I) or a pharmaceutically acceptable salt thereof in the manufacture of a medicament for treating duodenal or gastric ulcer relapse.

## Patentansprüche

1. Pharmazeutische Zusammensetzung enthaltend ein helicobacter-hemmendes antimikrobielles Mittel und eine Verbindung der Formel (I): 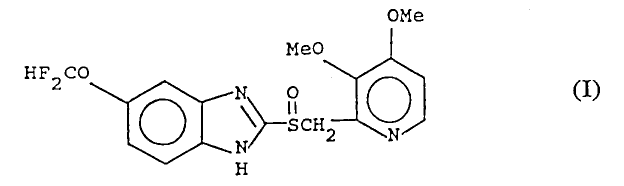 oder ein pharmazeutisch verträgliches Salz davon, und eine pharmazeutisch verträgliche Trägersubstanz.

2. Pharmazeutische Zusammensetzung gemäß Anspruch 1, in der die Verbindung der Formel (I) in Form ihres Natriumsalzes vorliegt.

3. Pharmazeutische Zusammensetzung gemäß Anspruch 2, in der das helicobacter-hemmende antimikrobielle Mittel Amoxicillin ist.

4. Pharmazeutische Zusammensetzung gemäß Anspruch 2, in der das helicobacter-hemmende antimikrobielle Mittel Amoxicillin und Metronidazol ist.

5. Pharmazeutische Zusammensetzung gemäß einem der Ansprüche 2-4, in der das helicobacter-hemmende antimikrobielle Mittel zusätzlich ein pharmazeutisch verträgliches Wismutsalz beinhaltet.

6. Pharmazeutische Zusammensetzung gemäß Anspruch 1, in der das helicobacter-hemmende antimikrobielle Mittel ein pharmazeutisch verträgliches Wismutsalz ist.

7. Pharmazeutische Zusammensetzung gemäß einem der Ansprüche 1-6 zur Anwendung in der therapeutischen Behandlung.

8. Pharmazeutische Zusammensetzung gemäß einem der Ansprüche 1-6 zur Anwendung in der Behandlung oder Verhütung gastrointestinaler Funktionsstörungen.

9. Verwendung eines helicobacter-hemmenden antimikrobiellen Mittels und einer Verbindung der Formel (I) oder eines pharmazeutisch verträglichen Salzes davon zur Herstellung eines Arzneimittels für die Behandlung oder Verhütung gastrointestinaler Funktionsstörungen.

10. Verwendung eines helicobacter-hemmenden antimikrobiellen Mittels und einer Verbindung der Formel (I) oder eines pharmazeutisch verträglichen Salzes davon zur Herstellung eines Arzneimittels für die Behandlung rezidivierender Duodenal- oder Magengeschwüre.

## Revendications

1. Composition pharmaceutique contenant un agent antimicrobien inhibant les Helicobacter et un composé de formule (I) : 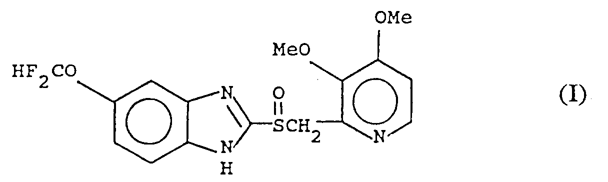 ou un sel d'un tel composé, admissible en pharmacie, ainsi qu'un véhicule admissible en pharmacie.

2. Composition pharmaceutique conforme à la revendication 1, dans laquelle le composé de formule (I) se trouve sous la forme de son sel de sodium.

3. Composition pharmaceutique conforme à la revendication 2, dans laquelle l'agent antimicrobien inhibant les Helicobacter est de l'amoxicilline.

4. Composition pharmaceutique conforme à la revendication 1, dans laquelle l'agent antimicrobien inhibant les Helicobacter est de l'amoxicilline et du métronidazole.

5. Composition pharmaceutique conforme à l'une des revendications 2 à 4, dans laquelle l'agent antimicrobien inhibant les Helicobacter contient en outre un sel de bismuth admissible en pharmacie.

6. Composition pharmaceutique conforme à la revendication 1, dans laquelle l'agent antimicrobien inhibant les Helicobacter est un sel de bismuth admissible en pharmacie.

7. Composition pharmaceutique conforme à l'une des revendications 1 à 6, destinée à être utilisée en thérapeutique.

8. Composition pharmaceutique conforme à l'une des revendications 1 à 6, destinée à être utilisée pour traiter ou prévenir des troubles gastro-intestinaux.

9. Emploi d'un agent antimicrobien inhibant les Helicobacter et d'un composé de formule (I) ou d'un sel d'un tel composé, admissible en pharmacie, dans la préparation d'un médicament destiné au traitement ou à la prévention des troubles gastro-intestinaux.

10. Emploi d'un agent antimicrobien inhibant les Helicobacter et d'un composé de formule (I) ou d'un sel d'un tel composé, admissible en pharmacie, dans la préparation d'un médicament destiné au traitement des récidives d'ulcères gastriques ou duodénaux.
